# EUROPÄISCHE PATENTSCHRIFT

(11) **EP 0 734 437 B1**
(45) Veröffentlichungstag und Bekanntmachung des Hinweises auf die Patenterteilung: **17.11.1999**
(21) Anmeldenummer: 95904461.1
(22) Anmeldetag: 13.12.1994
(51) Int. Cl.: C12N 11/14, C12N 11/18, C12N 9/06, C12N 9/84

(54) **TRÄGERFIXIERTE PENICILLIN-G-AMIDASE, GLUTARYL-7-ACA-ACYLASE ODER D-AMINOSAÜRE-OXIDASE**
SUBSTRATE-FIXED PENICILLIN G AMIDASE, GLUTARYL-7-ACA ACYLASE OR D-AMINOACID OXIDASE
PENICILLINE G-AMIDASE, GLUTARYL-7-ACA-ACYLASE OU OXYDASE D'AMINOACIDE-D FIXEE A UN SUPPORT

(30) Priorität: 15.12.1993 DE 4342770
(43) Veröffentlichungstag der Anmeldung: 02.10.1996
(73) Patentinhaber: Roche Diagnostics GmbH, 68305 Mannheim (DE)
(72) Erfinder: WEDEKIND, Frank, D-82377 Penzberg (DE); DASER, Adelheid, D-82444 Schlehdorf (DE); TISCHER, Wilhelm, D-82380 Peissenberg (DE)
(74) Vertreter: Weiss, Wolfgang, Dipl.-Chem. Dr.
(86) Internationale Anmeldenummer: EP9404132
(87) Internationale Veröffentlichungsnummer: WO9516773

(56) Entgegenhaltungen:
- EP-A- 0 231 093
- EP-A- 0 492 495
- EP-A- 0 496 993
- INDIAN JOURNAL OF CHEMISTRY, Bd.32B, Januar 1993, NEW DELHI Seiten 40 - 43 DANZIG, J. ET AL 'Bioreaction engineering for improved 6-APA production'

## Beschreibung

Die vorliegende Erfindung betrifft trägerfixierte Enzyme, ausgewählt aus der Gruppe, bestehend aus Penicillin-G-Amidase, Glutaryl-7-ACA-Acylase und D-Aminosäureoxidase, die Verwendung dieser Enzyme bei einer enzymatischen Synthesereaktion sowie ein Verfahren zur Verbesserung der Volumenaktivität und Stabilität dieser trägerfixierten Enzyme.

Die Fixierung biologisch aktiver Substanzen, z.B. Enzyme, an feste Trägermaterialien ist durch eine Vielzahl von Methoden möglich und in vielen Monographien und Veröffentlichungen beschrieben (siehe z.B. "Characterization of Immobilized Biocatalysts" Buchholz, K., (Herausgeber), DECHEMA Monographs Nr. 1724-1731, Vol. 84 (1979), "Protein Immobilization - Fundamentals and Applications" Taylor R.F. (Herausgeber), Marcel Dekker, Inc. (1991)). Neben nicht-kovalenten Immobilisierungstechniken, wie etwa Adsorption, Einschluß, Mikroverkapselung, Chelatierung und Aggregation, haben sich für industrielle Anwendungen, insbesondere kovalente Fixierungsmethoden durchgesetzt.

Als Trägermaterialien zur Fixierung biologisch aktiver Substanzen sind sowohl organische als auch anorganische Materialien auf synthetischer und natürlicher Basis bekannt. Die kovalente Fixierung von Proteinen an Trägermaterialien erfolgt im allgemeinen durch eine Kupplung der Proteine über die reaktiven Seitenketten ihrer Aminosäuren an den Träger. In der Regel ist hierzu eine chemische Aktivierung des Trägers oder/und des Proteins erforderlich. Die Kupplungschemie wird dabei jeweils von der Art des Proteins sowie von der verwendeten Trägermatrix bestimmt. Aufgrund der ganz erheblichen Unterschiede in den Sekundär-, Tertiär- und Quartärstrukturen von Proteinen ist die Eignung spezifischer Trägermaterialien für spezifische Enzyme grundsätzlich nicht vorhersagbar.

Für die meisten kommerziellen Anwendungen zur Fixierung biologisch aktiver Substanzen haben sich organische Trägermaterialien durchgesetzt, in die eine Vielzahl von reaktiven Gruppen eingeführt werden kann. Beispiele hier für sind natürliche Polymere, wie etwa Polysaccharidderivate und Strukturproteine sowie synthetische makromolekulare Substanzen auf Basis von Polystyrol oder Polyacrylat. Diese organischen Trägermaterialien sind durch gängige chemische Verfahren aktivierbar oder verfügen schon über reaktive Gruppen, die Verknüpfungen mit einem zu immobilisierenden Protein zulassen. Diese organischen Trägermaterialien zeigen jedoch noch einige Nachteile, die ihre Verwendbarkeit einschränken. So sind natürliche Polysaccharidderivate oft empfindlich gegenüber mikrobiellem Abbau (Cellulose), besitzen ungünstige Partikeleigenschaften (Cellulose, Fasern unterschiedlicher Länge) sowie schlechte mechanische oder/und Quellungseigenschaften.

Synthetische Materialien sind in der Regel gegenüber einem mikrobiellen Angriff unempfindlich, sie weisen jedoch andere Nachteile auf. Trägermaterialien auf Polyacrylamidbasis, die meist in Protein-Monomer-Coimmobilisierungen eingesetzt werden, sind aus potentiell kanzerogenen Monomeren (Acrylamid) aufgebaut und zeigen starke Quellung wäßrigen Systemen. Polyacrylate, Polymethacrylate, Hydroxyalkylmethacrylate sowie Polymethacrylamide können durch Polymerisation der entsprechenden Monomere mit geeigneten Vernetzern hergestellt werden und sind teilweise kommerziell erhältlich. Insbesondere durch Verwendung von Glycidylderivaten bei Copolymerisationen oder nachträglichen Modifizierungen lassen sich mit diesen Materialien bereits aktivierte Träger herstellen (z.B. Eupergit, Biosynth). Diese Trägermaterialien zeigen eine relativ starke Quellung und bei Immobilisierung von Enzymen werden oft nur geringe Volumenaktivitäten erreicht, wodurch die Anwendung dieser Trägermaterialien bei enyzmatischen Synthesereaktionen weniger geeignet ist.

Anorganische Träger zeigen vielfach günstigere mechanische Eigenschaften, thermische Stabilität, Beständigkeit gegenüber organischen Lösungsmitteln und mikrobiellem Angriff als organische Träger. Beispiele für anorganische Träger sind Mineralien, wie etwa Bentonit, Attapulgit, Diatomeenerde. Sie zeigen oft eine weite Porengrößenverteilung. Nicht-poröse Materialien, wie Metalle oder Metalloxide besitzen in der Regel nur geringe Bindungsoberflächen und sind daher zur Immobilisierung von biologischen Substanzen im allgemeinen ungeeignet. Controlled Pore Glass (CPG) läßt sich zwar mit definierten Porengrößen und Bindungsoberflächen herstellen, es ist jedoch schon unter moderaten alkalischen Bedingungen (pH > 8) instabil. Alle bisher erwähnten anorganischen Materialien müssen überdies vor der eigentlichen Kupplungsreaktion in geeigneter Weise vorbehandelt und in ein aktiviertes Derivat überführt werden. Diese Derivatisierung ist aufgrund der inerten Natur der Träger oft relativ schwierig. Am häufigsten erfolgt hierzu eine Beschichtung des Trägers durch Silanisierung. Dann wird das zu immobilisierende Protein nach der Funktionalisierung des Trägers im allgemeinen mit 3-Aminopropylethoxysilan kovalent gebunden. Auf diese Weise erhält man jedoch im allgemeinen nur geringe Kupplungsausbeuten des Proteins.

Der Einsatz organofunktionalisierter Polysiloxane als Enzymträger ist bekannt (siehe z.B. Poster Nr. 518 und 519, DECHEMA-Tagung der Biotechnologen 1992; Poster 5.218 DECHEMA-Jahrestagung der Biotechnologen 1993). Dort wird beschrieben, daß organofunktionelle Polysiloxane, insbesondere aminofunktionelle Polysiloxane, als Trägermaterialien zur Immobilisierung der Enzyme Invertase, Lactase und Glucoseoxidase verwendet werden können. Für diese Enzyme wird eine hohe Bindungskapazität und - effektivität sowie eine erhöhte Stabilität beschrieben. Eine Erhöhung der Volumenaktivität der immobilisierten Enzyme gegenüber anderen Trägermaterialien wird nicht offenbart.

Die Enzyme Penicillin-G-Amidase, Glutaryl-7-ACA-Acylase und D-Aminosäureoxidase werden zur industriellen Herstellung modifizierter Penicillin- und Cephalosporin-Antibiotika verwendet. D-Aminosäureoxidase und Glutaryl-7-ACA-Acylase katalysieren eine zweistufige enzymatische Umsetzung von Cephalosporin-C oder Derivaten und Salzen davon zu 7-Amino-Cephalosporansäure (7-ACA) oder Derivaten davon (siehe z.B. EP-B-0 211 033, EP-A-0 409 521). Penicillin-G-Amidase katalysiert die Synthese von Cefalotin aus 7-ACA und Thienylessigsäure.

Bei Durchführung dieser obigen Reaktionen im industriellen Maßstab werden die genannten Enzyme meist in immobilisierter Form verwendet. EP-B-0 211 033 beschreibt z.B. die Anreicherung von D-Aminosäureoxidase aus dem Mikroorganismus Trigonopsis variabilis und die Immobilisierung dieses Enzyms an CNBr-aktivierte Sepharose, wobei eine Volumenaktivität von 7 U/ml Trägermaterial erhalten wird.

EP-A-0 492 495 offenbart die Immobilisierung von D-Aminosäure-oxidase an ein Copolymerisat, das im wesentlichen aus Vinylacetat- und/oder Vinylalkoholeinheiten und Einheiten eines Vernetzungsmittels besteht. Es wird eine Volumenaktivität von 32 U/g feuchter Träger erhalten. Das immobilisierte Enzym ist bei 30°C für 6 Monate stabil. Bei Verwendung von anderen festen Trägern (BrCN-aktivierte Sepharose®, Vinylsepharose und Eupergit®) werden geringere Volumenaktivitäten und Stabilitäten erhalten.

EP-A-O 496 993 beschreibt die Immobilisierung von D-Aminosäure-oxidase aus Rhodotorula gracilis und Glutaryl-7-ACA-Acylase aus Acinetobacter species, die in transformierten E.coli-Zellen erzeugt wurde, auf eine Reihe fester Träger, z.B. stark basische Polystyrolharze mit einer quaternären Aminfunktionalität, wie etwa Amberlite® IFA900,, schwach basische Polystyrolharze mit einer primären Aminofunktionalität, wie etwa Duolite® A365, mittelbasische polykondensierte Phenolformaldehydharze mit sekundären und tertiären Aminfunktionalitäten, wie etwa Duolite® A568 oder Duolite® A7. Für die D-Aminosäureoxidase wird eine Volumenaktivität von bis zu maximal 75 U/g feuchter Träger (Epoxid-funktionalisiertes Eupergit® C) beschrieben. Für Glutaryl-7-ACA-Acylase wird eine maximale Volumenaktivität von 41 U/g feuchter Träger (Epoxid-funktionalisiertes Eupergit® C) beschrieben.

WO90/12110 offenbart die Immobilisierung von D-Aminosäureoxidase und Glutaryl-7-ACA-Acylase an einen Siliciumoxid-Polyvinylchlorid-Verbundstoff in Blattform, der mit Polyethylenimin und Glutaraldehyd derivatisiert werden kann. Eine Erhöhung der Volumenaktivität durch diese Art der Immobilisierung gegenüber anderen Trägermaterialien wird nicht beschrieben.

Die Literaturstellen Wood et al. (J. Biotech. 13 (1990), 305-314) und Cobbs et al. (Biotechnology Techniques, Vol. 4, Nr. 1 (1990), 5-10) beschreiben die Immobilisierung von D-Aminosäure-oxidase an einen festen Träger mit Hilfe eines trifunktionellen Aziridins. Eine Erhöhung der Volumenaktivität durch diese Art der Immobilisierung gegenüber anderen Trägermaterialien wird nicht beschrieben.

Die Literaturstelle Szwajcer-Dey et al. (Appl. Biochem. Biotech. 27 (1991), 239-250) beschreibt die Immobilisierung von D-Aminosäureoxidase aus Trigonopsis variabilis auf Metalloxidteilchen und auf CNBr-aktivierte Sepharose. Es wird eine Immobilisierung von 23 mg D-Aminosäureoxidase pro g feuchte Sepharose beschrieben. Eine Erhöhung der Volumenaktivität durch Verwendung von Metalloxidträgerteilchen wird nicht offenbart.

Die der vorliegenden Erfindung zugrundeliegende Aufgabe bestand somit darin, neue trägerfixierte Enzyme, ausgewählt aus der Gruppe, bestehend aus Penicillin-G-Amidase, Glutaryl-7-ACA-Acylase und D-Aminosäureoxidase bereitzustellen, die gegenüber bekannten trägerfixierten Enzymen eine höhere Volumenaktivität oder/und Stabilität aufweisen. Eine weitere Aufgabe der vorliegenden Erfindung bestand darin, ein einheitliches Trägersystem für die drei oben genannten Enzyme bereitzustellen, wodurch ein gleichzeitiger Einsatz dieser Enzyme in enzymatischen Synthesereaktoren erheblich vereinfacht wird.

Die erfindungsgemäße Aufgabe wird gelöst durch die Bereitstellung eines trägerfixierten Enzyms, ausgewählt aus der Gruppe, bestehend aus Penicillin-G-Amidase (E.C. 3.5.1.11), Glutaryl-7-ACA-Acylase und D-Aminosäure-Oxidase (E.C.1.4.3.3), das durch kovalente Bindung an ein aminofunktionelles Organosiloxanpolymer-Trägermaterial fixiert ist.

Der Begriff "aminofunktionelles Organosiloxanpolymer" gemäß vorliegender Erfindung umfaßt polymere Verbindungen, in denen Siliciumatome und gegebenenfalls Titan-, Zirkonium- oder/und Aluminiumatome über Sauerstoffatome verknüpft sind und freie Valenzen dieser Metallatome durch organische Reste abgesättigt sind, die aktivierbare Aminogruppen enthalten. Die Aminogruppen sind vorzugsweise primäre Aminogruppen. Derartige aminofunktionelle Organosiloxane sind kommerziell erhältlich, z.B. von der DEGUSSA AG, Frankfurt, Bundesrepublik Deutschland, unter der Bezeichnung DELOXAN®. Die Zusammensetzung und Herstellung bevorzugter aminofunktioneller Organopolysiloxane ist z.B. in DE-OS 31 20 214, DE-OS 38 37 418, DE-OS 39 25 359 und DE-OS 39 25 360 beschrieben, auf deren Offenbarung hiermit Bezug genommen wird.

Beispiele für geeignete funktionelle Aminogruppen auf den Organosiloxanpolymeren sind z.B. -NH₂, -RNH₂, -NH-R-NH₂ oder -R-(NH-R')ₙ-NH₂, wobei R und R' z.B. unabhängig voneinander eine lineare oder verzweigte Alkylengruppe mit 1 bis 10 C-Atomen, eine Cycloalkylengruppe mit 5 bis 8 C-Atomen oder eine lineare oder verzweigte Alkylengruppe, die eine Cyclohexylen- oder Phenylen-einheit enthält, sein können. Besonders bevorzugte Beispiele für funktionelle Aminogruppen sind -NH₂, -NH(CH₂)₂-NH₂, -(CH₂)₃-NH₂ oder-(CH₂)₂-NH-(CH₂)₂-NH-(CH₂)₂-NH₂.

Die Gestalt bzw. Form des aminofunktionellen Trägermaterials kann an sich beliebig sein. Bevorzugt sind jedoch partikelförmige Trägermaterialien mit einer geeigneten Größe, die einerseits eine hohe spezifische Oberfläche aufweisen und andererseits eine gute Handhabbarkeit ermöglichen. Ein bevorzugter mittlerer Durchmesserbereich der Partikel ist von 0,01 bis 3 mm. Vorzugsweise ist das Trägermaterial im wesentlichen kugelförmig. Die Herstellung kugelförmiger aminofunktioneller Organosiloxanpolymere ist in den oben genannten DE-OS 39 25 359 und DE-OS 39 25 360 beschrieben, auf deren Offenbarung hiermit Bezug genommen wird. Eine mittlere Partikelgröße des Trägermaterials von 0,05 bis 1 mm ist bevorzugt und eine mittlere Partikelgröße von 0,1 bis 0,4 mm ist besonders bevorzugt. Am meisten bevorzugt ist für Penicillin-G-Amidase eine mittlere Partikelgröße von 0,1 bis 0,3 mm und für Glutaryl-7-ACA-Acylase und D-Aminosäureoxidase von 0,2 bis 0,4 mm.

Die Kapazität (d.h. die Anzahl der Aminogruppen) des zur Fixierung der Enzyme verwendeten aminofunktionellen Trägers beträgt vorzugsweise 0,5 - 5 mval/g Träger, besonders bevorzugt 1 - 4 mval/g Träger.

Für die Fixierung der Enzyme an den aminofunktionellen Träger können verschiedene, aus dem Stand der Technik bekannte Methoden verwendet werden. Diese Methoden umfassen im allgemeinen eine Aktivierung der auf dem Träger befindlichen Aminogruppen durch ein Aktivierungsreagenz. Die aktivierten Gruppen auf dem Träger können anschließend mit reaktiven Gruppen des Enzyms, insbesondere mit Aminogrppen in der Seitenkette, reagieren. Auf diese Weise werden trägerfixierte Enzyme erhalten, die überraschenderweise eine gegenüber anderen Trägermaterialien erhöhte Volumenaktivität aufweisen.

Durch Reaktion mit Thiophosgen bzw. 1,4-Phenylendiisothiocyanat kann die Aminogruppe des Trägers in eine reaktive Isothiocyanatgruppe überführt werden. Durch Reaktion des Trägers mit Dianhydriden (z.B. Bernsteinsäureanhydrid) können Carboxylfunktionen eingeführt werden, die sich in reaktive Säurechloride und Ester (z.B. N-Hydroxysuccinimidester) überführen lassen. Weiterhin kann eine Aktivierung der Aminogruppe auf dem Träger durch Reaktion mit Chlortriazinen (z.B. Cyanurchlorid) erfolgen. Ein weiteres Beispiel für die Aktivierung ist eine Reaktion von primären Aminogruppen auf dem Träger mit 4-Nitrobenzoylchlorid und Diazotierung der aromatischen Aminogruppe und/oder Überführung ins entsprechende Hydrazin. Auch durch Reaktion mit Bis-Epoxiden (z.B. 1,4-Butandioldiglycidylether) oder Epichlorhydrin können die Aminogruppen durch Einführung von reaktiven Epoxidgruppen aktiviert werden. Einzelheiten derartiger und anderer Methoden sind z.B. in "Covalent and Coordination Immobilization of Proteins, Cabral J.M.S., Kennedy, J.F., in Protein Immobilization, Fundamentals and Application (Taylor R.F., Hrsg.) Marcel Dekker Inc., 1991, beschrieben.

Vorzugsweise wird die Aktivierung der Aminogruppen auf dem Träger jedoch durch Umsetzung mit Dialdehyden (z.B. Glutardialdehyd) durchgeführt. Diese Methode ist besonders leicht auch in einem wäßrigen Medium durchzuführen. Damit kann die Verwendung toxischer Verbindungen und organischer Lösungsmittel vermieden werden.

Nach der Aktivierung der Aminogruppen auf dem Träger erfolgt die Fixierung des Enzyms durch Kontaktieren des aktivierten Träger mit dem Enzym unter geeigneten Bedingungen. Anschließend können nicht umgesetzte reaktive Gruppen des Trägers abgesättigt werden. Bei einem Aldehyd-aktivierten Träger kann die Absättigung z.B. mit Ethanolamin erfolgen.

In einer Ausführungsform betrifft die vorliegende Erfindung eine trägerfixierte Penicillin-G-Amidase. Die Penicillin-G-Amidase ist ein Enzym, das aus einer Vielzahl von unterschiedlichen Bakterienspezies und einigen Pilzen und Hefen, vorzugsweise aus E.coli erhältlich ist. Vorzugsweise ist das Gewichtsverhältnis von Penicillin-G-Amidase zu Trägermaterial im Bereich von 2 bis 200 mg Protein pro g feuchter Träger, besonders bevorzugt im Bereich von 40 bis 80 mg Protein pro g feuchter Träger. Bei der erfindungsgemäßen Immobilisierung von Penicillin-G-Amidase an aminofunktionelle Organosiloxanpolymere lassen sich beispielsweise spezifische Volumenaktivitäten im Bereich von mehr als 100 U/g feuchter Träger und insbesondere mehr als 125 U/g feuchter Träger erreichen. Vorzugsweise ist die spezifische Volumenaktivität im Bereich von 100 bis 300 U/g feuchter Träger und besonders bevorzugt im Bereich von 125 bis 275 U/g feuchter Träger. Die Einheit "U" für Penicillin-G-Amidase ist definiert als die Enzymmenge, die 1 µmol Penicillin G pro Minute unter Standardbedingungen (5 % Penicillin G, 0,6 mmol/l Kaliumphosphatpuffer pH 8,0; 28°C) hydrolysiert.

Eine weitere Ausführungsform der vorliegenden Erfindung betrifft eine trägerfixierte Glutaryl-7-ACA-Acylase. Die Glutaryl-7-ACA-Acylase ist z.B. aus Acinetobacter spp. oder mit dem Glutaryl-7-ACA-Acylase-Gen transformierten E.coli-Zellen (vgl. EP-A-0 496 993) erhältlich. Bei der trägerfixierten Glutaryl-7-ACA-Acylase ist das Gewichtsverhältnis von Enzym zu Trägermaterial vorzugsweise im Bereich von 10 bis 110 mg Protein pro g feuchter Träger, besonders bevorzugt im Bereich von 20 bis 70 mg Protein pro g feuchter Träger. Die spezifische Volumenaktivität einer erfindungsgemäß immobilisierten Glutaryl-7-ACA-Acylase kann mehr als 100 U/g feuchter Träger, insbesondere mehr als 125 U/g feuchter Träger betragen. Vorzugsweise liegt die spezifische Volumenaktivität der trägerfixierten Glutaryl-7-ACA-Acylase im Bereich von 100 bis 250 U/g feuchter Träger, besonders bevorzugt im Bereich von 125 bis 200 U/g feuchter Träger. Die Einheit "U" für Glutaryl-7-ACA-Acylase ist definiert als die Enzymmenge, die 1 µmol 7β-(4-Carboxybutanamido)-cephalosporansäure (Glutaryl-7-ACA) pro Minute unter Standardbedingungen (2 % Gl-7-ACA, 5 mmol/l Kaliumphosphatpuffer pH 8,0; 37°C) hydrolysiert.

Noch eine weitere Ausführungsform der vorliegenden Erfindung betrifft trägerfixierte D-Aminosäureoxidase. Das Gewichtsverhältnis von trägerfixierter D-Aminosäureoxidase zu Trägermaterial ist vorzugsweise im Bereich von 5 bis 25 mg Protein pro g feuchter Träger, besonders bevorzugt im Bereich von 8 bis 15 mg Protein pro g feuchter Träger. Die spezifische Volumenaktivität der D-Aminosäureoxidase kann bei erfindungsgemäßen Immobilisierung 100 U/g feuchter Träger oder mehr, insbesondere 125 U/g feuchter Träger oder mehr betragen. Vorzugsweise liegt die spezifische Volumenaktivität im Bereich von 100 bis 200 U/g, besonders bevorzugt im Bereich von 125 bis 175 U/g feuchter Träger. Die Einheit "U" für D-Aminosäureoxidase ist in diesem Zusammenhang als Cephalosporin C-(CephC-) Einheit definiert. Die Ceph-C-Aktivität wird in Anwesenheit von Katalase/O₂/H₂O₂ bestimmt. Die gebildeten Mengen an Produkten (α-Ketoadipinyl-7-aminocephalosporansäure und Glutaryl-7-aminocephalosporansäure) werden nach Trennung der Reaktionslösung durch HPLC quantifiziert. Die Aktivität von 1 U entspricht der Bildung von 1 µmol Produkte/min bei 25°C unter Standardbedingungen (1,3 kU Katalase, O₂-gesättigte Lösung, 0,007 % H₂O₂).

Gegebenenfalls kann die D-Aminosäureoxidase auch mit Katalase (E.C. 1.11.1.6) coimmobilisiert vorliegen. In diesem Fall werden im allgemeinen 50 U - 100 U Katalase pro U (Ceph-C-U) D-Aminosäureoxidase verwendet. Die Immobilisierung der Katalase erfolgt entsprechend der oben beschriebenen Techniken. Die Aktivität von 1 U Katalase entspricht der Enzymmenge, die 1 µmol H₂O₂ pro Minute unter Standardbedingungen (25 mmol/l Kaliumphosphatpuffer pH 7,0; 0,018 % H₂O₂) hydrolysiert.

Die erfindungsgemäße D-Aminosäureoxidase ist aus einer großen Anzahl von Organismen erhältlich, z.B. E.coli, Pseudomonas spezies, Aerobacter spezies, Candida tropicalis, Penicillinium roqueforti, Aspergillus flavus und Aspergillus niger, Neurospora crassa, Nocardia, Citrobacter, Rhodotorula und Trigonopsis variabilis. Die D-Aminosäureoxidase aus Trigonopsis variabilis ist besonders bevorzugt. Die Isolierung und Reinigung dieses Enzyms ist z.B. in EP-A-0 211 033 beschrieben.

Ein weiterer Gegenstand der vorliegenden Erfindung ist die Verwendung der erfindungsgemäß trägerfixierten Enzyme einzeln oder in Kombinationen von mindestens zwei der genannten Enzyme bei einer enzymatischen Synthesereaktion. Penicillin-G-Amidase kann beispielsweise bei der Synthese von Cephalothin aus 7-ACA und Thienylessigsäure verwendet werden. D-Aminosäureoxidase und Glutaryl-7-ACA-Acylase können bei der zweistufigen enzymatischen Synthese von 7-ACA aus Cephalosporin C verwendet werden.

Die erfindungsgemäßen trägerfixierten Enzyme unterscheiden sich von den bekannten trägerfixierten Enzymen durch erhöhte Volumenaktivität, erhöhte Stabilität oder/und verbesserte mechanische Eigenschaften des Immobilisats. Ein weiterer Gegenstand der vorliegenden Erfindung ist somit ein Verfahren zur Verbesserung der Volumenaktivität von trägerfixierten Enzymen, ausgewählt aus der Gruppe, bestehend aus Penicillin-G-Amidase (E.C. 3.5.1.11), Glutamyl-7-ACA-Acylase und D-Aminosäure-Oxidase (E.C.1.4.3.3), welches dadurch gekennzeichnet ist, daß man das Enzym durch kovalente Bindung an ein aminofunktionelles Organosiloxanpolymer-Trägermaterial fixiert.

Wie bereits oben ausgeführt, erfolgt die Fixierung der Enzyme an das Trägermaterial vorzugsweise über einen Dialdehyd, z.B. Glutardialdehyd, aber auch alle anderen zur Immobilisierung von Proteinen an aminofunktionelle Träger geeigneten Verfahren können verwendet werden.

Überraschenderweise zeigen die erfindungsgemäßen trägerfixierten Enzyme hohe Volumenaktivitäten, wodurch hohe Produktausbeuten in industriellen Synthesereaktionen erreicht werden. Insbesondere eignen sich die erfindungsgemäßen immobilisierten Enzyme in Reaktionen, bei denen niedrige Temperaturen, z.B. 4 - 30°C, herrschen oder/und organische Lösungsmittel, z.B. Dimethylformamid, Dimethylsulfoxid etc., vorhanden sind. Weiterhin zeigen die erfindungsgemäß immobilisierten Enzyme verbesserte mechanische Eigenschaften gegenüber anderen Trägermaterialien, wie etwa Säulengängigkeit und Kompressibilität.

Die vorliegende Erfindung soll weiterhin durch die folgenden Beispiele in Verbindung mit den Figuren 1 bis 5 veranschaulicht werden. Es zeigen:
Figur 1 einen Vergleich der spezifischen Volumenaktivitäten von Penicillin-G-Amidase, die an ein aminofunktionalisiertes Organopolysiloxan oder an Polymethacrylat fixiert wurde,
Figur 2 einen Vergleich der Produktausbeute bei einer Cephalothinsynthese mit immobilisierter Penicillin-G-Amidase, die an ein aminofunktionalisiertes Organopolysiloxan oder an Polymethacrylat fixiert wurde,
Figur 3 einen Vergleich der Volumenaktivitäten von immobilisierter Glutaryl-7-ACA-Acylase bei verschiedenen Trägermaterialien,
Figur 4 einen Vergleich der Durchflußeigenschaften verschiedener Säulenmaterialien und
Figur 5 einen Vergleich der Kompressibilität verschiedener Säulenmaterialien.

### BEISPIELE

### Beispiel 1

### Herstellung von immobilisierter Penicillin-G-Amidase

Aminofunktionalisierte Organopolysiloxane wurden in verschiedenen Spezifikationen von der DEGUSSA AG bezogen. Die funktionellen Gruppen der einzelnen Substanzen sind in der folgenden Tabelle 1 dargestellt:

**Tabelle 1**

| Material | Kapazität | | Partikelgröße | funktionelle Gruppe |
|---|---|---|---|---|
| | mval/g | mval/ml | mm | |
| PAP III | 3,14 | 0,66 | 0,1 - 0,3 | -NH₂ |
| DAP III | 2,77 | 0,49 | 0,1 - 0,3 | -NH-(CH2)2-NH2 |
| DAP IV | 1,7 | 0,34 | 0,2 - 0,4 | -NH-(CH2)2-NH2 |

Zu 1 g aminogruppenhaltigem PAP III Trägermaterial werden 1 ml einer 25 % Glutardialdehydlösung in 0,1 mol/l Kaliumphosphatpuffer pH 7,0 zugesetzt und 1 Stunde bei Raumtemperatur mit einem Propellerrührer gerührt. Das behandelte Gel wird mit 20 ml 0,1 mol/l Kaliumphosphatpufferlösung pH 7,0 gewaschen. 500 - 5000 U einer Penicillin-G-Amidaselösung in 10 ml 0,01 mol/l Kalium-phosphat pH 7,0 werden zu dem aktivierten Trägermaterial zugegeben und die Suspension 2 Stunden bei Raumtemperatur gerührt. Mit 1 ml 0,1 mol/l Ethanolaminlösung pH 9,0 werden überschüssige reaktive Gruppen durch einstündiges Rühren bei Raumtemperatur abgesättigt.

Im nachfolgenden wird bei 4°C zunächst mit 50 ml 0,02 mol/l Kaliumphosphatpuffer pH 7,0/1 mol/l NaCl, dann mit 50 ml 0,02 mol/l Kaliumphosphatpuffer gespült. Aktivitätsbestimmungen der Kupplungs- und Waschlösungen sowie des immobilisierten Enzyms werden unter pH-Stat-Bedingungen am Autotitrator durchgeführt (5 % PenG, 28°C, pH 8,0, 0,6 mol/l Kaliumphosphatpuffer). Die Angabe der spezifischen Aktivität erfolgt auf Basis des Feuchtgewichtes des Katalysators.

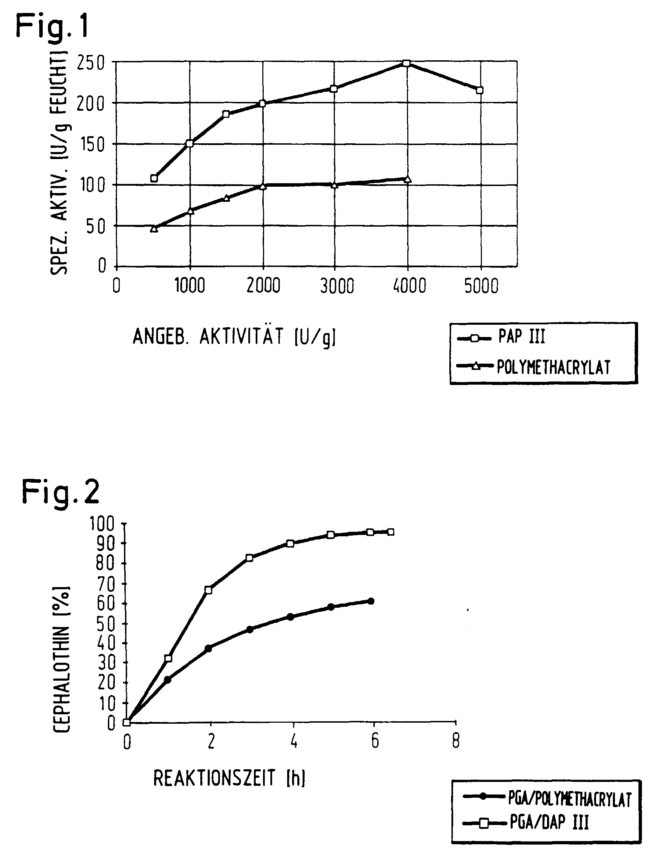

Gegenübergestellt in Figur 1 sind die spezifischen Volumenaktivitäten von PAP III und Polymethacrylat-Derivaten.

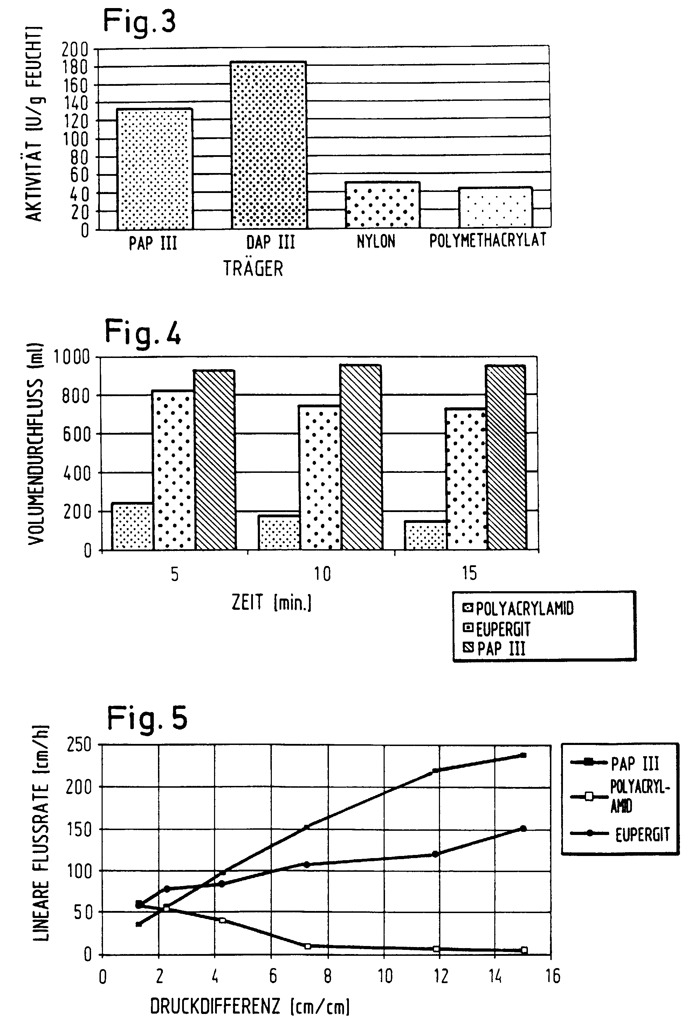

Die bei den erfindungsgemäßen Trägern erzielbaren hohen Volumenaktivitäten ermöglichen den Einsatz der immobilisierten Penicillin-G-Amidase (PGA) in Synthesereaktionen, bei denen niedrige Temperaturen und pH-Werte sowie organische Lösungsmittel für hohe Produktausbeuten erforderlich sind. Gegenübergestellt sind in Figur 2 Gleichgewichtssynthesen von Cefalothin aus 7-ACA und Thienylessigsäure bei volumengleichem Einsatz von immobilisierter PGA unter Verwendung von PAPIII- und Polymethylacrylatimmobilisierter PGA.

### Beispiel 2

### Immobilisierung von Glutaryl-7-ACA-Acylase

Die Fixierung erfolgt analog der PGA-Immobilisierung. Es werden 180 - 250 U/g feuchtes Trägermaterial DAP III bzw. PAP III eingesetzt. Die resultierenden Volumenaktivitäten sind in Figur 3 im Vergleich mit Polymethacrylat- und Nylonimmobilisaten dargestellt.

### Beispiel 3

### Immobilisierung von D-Aminosäureoxidase (DAO)

Das Enzym wird vor der Fixierung gegen 20 mmol/l KPO₄ Puffer pH 7,0/1 mmol/l Dithiothreitol dialysiert. 2500 U DAO (D-Alanin Units) werden pro g feuchtes Trägermaterial DAP III angeboten. Eine Blockierung der überschüssigen reaktiven Stellen erfolgt durch 1 Stunde Rühren mit 0,1 mol/l Ethanolaminlösung pH 8,0. Das Waschen erfolgt mit 0,5 mol/l NaCl/0,2 mmol/l DTT/0,5 mmol/l EDTA in 20 mmol/l KPO_{4,} pH 7,0. Es werden Aktivitäten > 1000 U/g feuchtes Trägermaterial erzielt.

Tabelle 2 zeigt die Volumenaktivitäten von immobilisierter DAO mit verschiedenen Trägermaterialien.

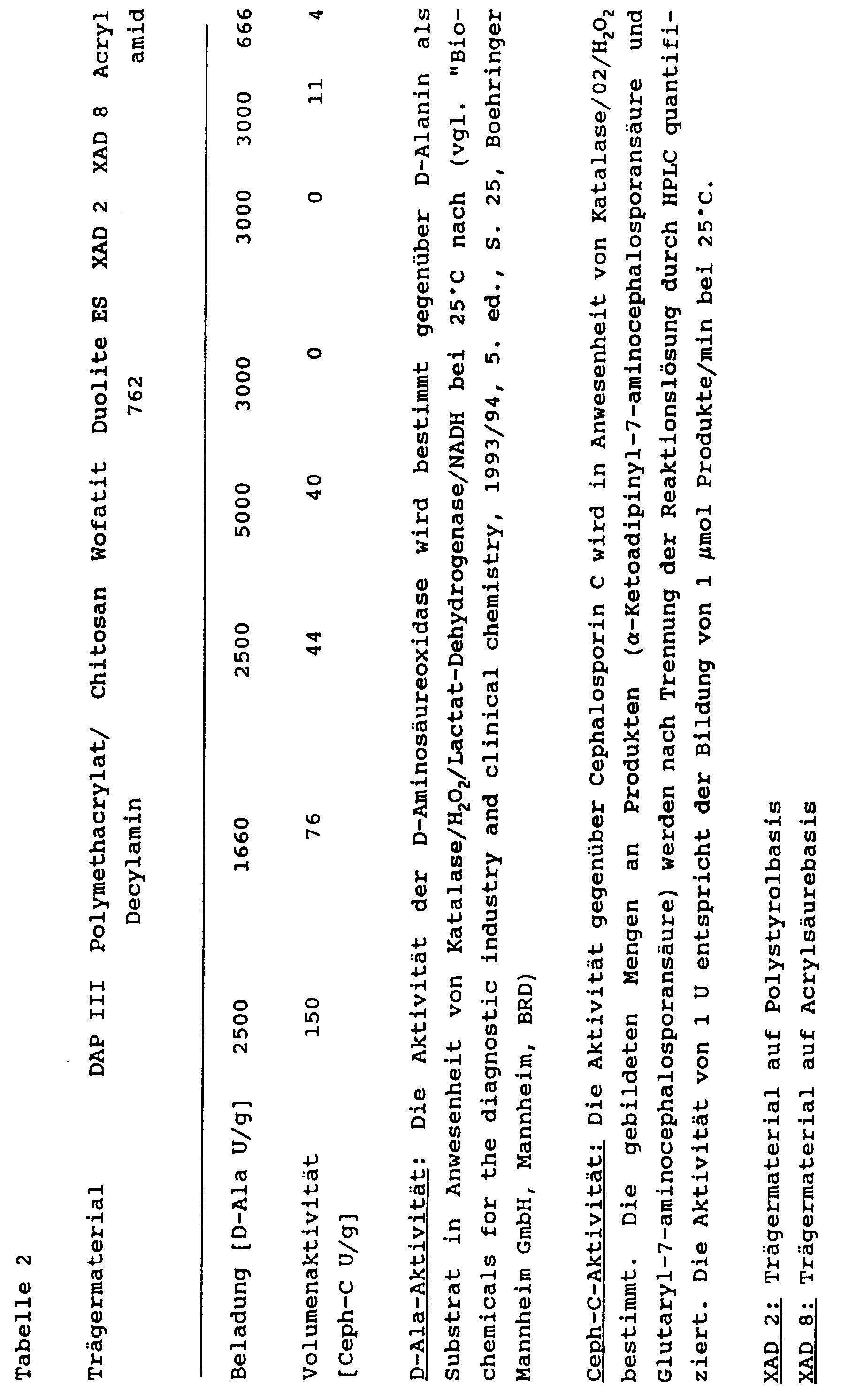

### Beispiel 4

### Mechanische Eigenschaften

### 1. Durchflußeigenschaften/Säulengängigkeit

Die Filtrationseigenschaften verschiedener Trägerkatalysatoren (Eupergit C, Polyacrylamidgel, PAP III) werden durch Bestimmung des Durchflußvolumens/Zeiteinheit bei konstantem hydrostatischem Druck und identischer Gelbetthöhe ermittelt. Der Versuch wird ohne eine vorherige Gelentspannung zweimal wiederholt. Das Ergebnis dieses Versuchs ist in Figur 4 gezeigt. Es ist ersichtlich, daß das aminofunktionelle Polysiloxan bessere Durchflußeigenschaften als die anderen Trägermaterialien besitzt.

### 2. Kompressibilität

Die Kompressibilität von Organopolysiloxan PAP III sowie Eupergit und Polyacrylamid wurde in einen Säulendurchflußtest bei Einsatz gleicher Gelbettvolumina und Füllhöhen ermittelt. Dargestellt ist die Abhängigkeit der linearen Flußrate vom hydrostatischen Druck in Figur 5. Es ist ersichtlich, daß das aminofunktionelle Polysiloxan bessere Kompressibilitätseigenschaften als die anderen Trägermaterialien besitzt.

## Patentansprüche

1. Trägerfixiertes Enzym, ausgewählt aus der Gruppe, bestehend aus Penicillin-G-Amidase (E.C. 3.5.1.11), Glutaryl-7-ACA-Acylase und D-Aminosäure-Oxidase (E.C.1.4.3.3), das durch kovalente Bindung an ein aminofunktionelles Organosiloxanpolymer-Trägermaterial fixiert ist.

2. Trägerfixiertes Enzym nach Anspruch 1,
**dadurch gekennzeichnet,**
daß das Enzym an das Trägermaterial über einen Dialdehyd fixiert ist.

3. Trägerfixiertes Enzym nach Anspruch 1 oder 2,
**dadurch gekennzeichnet,**
daß das Gewichtsverhältnis von Enzym zu Trägermaterial im Bereich von 1 bis 300 mg Protein pro g feuchter Träger ist.

4. Trägerfixiertes Enzym nach einem der Ansprüche 1 bis 3,
**dadurch gekennzeichnet,**
daß das Trägermaterial partikelförmig ist und einen mittleren Partikeldurchmesser von 0,01 bis 3 mm aufweist.

5. Trägerfixierte Glutaryl-7-ACA-Acylase nach einem der Ansprüche 1 bis 4,
**dadurch gekennzeichnet,**
daß das Gewichtsverhältnis von Enzym zu Trägermaterial im Bereich von 10 bis 110 mg Protein pro g feuchter Träger ist.

6. Trägerfixierte Glutaryl-7-ACA-Acylase nach Anspruch 5,
**dadurch gekennzeichnet,**
daß die spezifische Volumenaktivität im Bereich von 100 bis 250 U pro g feuchter Träger ist.

7. Trägerfixierte D-Aminosäure-Oxidase nach einem der Ansprüche 1 bis 4,
**dadurch gekennzeichnet,**
daß das Gewichtsverhältnis von Enzym zu Trägermaterial im Bereich von 5 bis 25 mg Protein pro g feuchter Träger ist.

8. Trägerfixierte D-Aminosäure-Oxidase nach Anspruch 7, die mit Katalase (E.C. 1.11.1.6) coimmobilisiert ist.

9. Verwendung von trägerfixierten Enzymen einzeln oder in Kombination nach einem der Ansprüche 1 bis 8 bei einer enzymatischen Synthesereaktion.

10. Verfahren zur Verbesserung der Volumenaktivität von trägerfixierten Enzymen, ausgewählt aus der Gruppe, bestehend aus Penicillin-G-Amidase (E.C. 3.5.1.11), Glutaryl-7-ACA-Acylase und D-Aminosäure-Oxidase (E.C.1.4.3.3),
**dadurch gekennzeichnet,**
daß man das Enzym durch kovalente Bindung an ein aminofunktionelles Organosiloxanpolymer-Trägermaterial fixiert.

## Claims

1. Enzyme immobilized on a carrier selected from the group comprising penicillin-G amidase (E.C. 3.5.1.11), glutaryl-7-ACA acylase and D-amino acid oxidase (E.C.1.4.3.3) which is immobilized by covalent binding to an aminofunctional organosiloxane polymer carrier material.

2. Enzyme immobilized on a carrier as claimed in claim 1,
**wherein**
the enzyme is immobilized on the carrier material by means of a dialdehyde.

3. Enzyme immobilized on a carrier as claimed in claim 1 or 2,
**wherein**
the weight ratio of enzyme to carrier material is in the range of 1 to 300 mg protein per g wet carrier.

4. Enzyme immobilized on a carrier as claimed in one of the claims 1 to 3,
**wherein**
the carrier material is particulate and has an average particle diameter of 0.01 to 3 mm.

5. Glutaryl-7-ACA acylase immobilized on a carrier as claimed in one of the claims 1 to 4,
**wherein**
the weight ratio of enzyme to carrier material is in the range of 10 to 110 mg protein per g wet carrier.

6. Glutaryl-7-ACA acylase immobilized on a carrier as claimed in claim 5,
**wherein**
the specific volume activity is in the range of 100 to 250 U per g wet carrier.

7. D-Amino acid oxidase immobilized on a carrier as claimed in one of the claims 1 to 4,
**wherein**
the weight ratio of enzyme to carrier material is in the range of 5 to 25 mg protein per g wet carrier.

8. D-Amino acid oxidase immobilized on a carrier as claimed in claim 7 which is co-immobilized with catalase (E.C.1.11.1.6).

9. Use of enzymes immobilized on carriers, individually or in combination, as claimed in one of the claims 1 to 8 in an enzymatic synthesis reaction.

10. Process for improving the volume activity of enzymes immobilized on carriers selected from the group comprising penicillin-G amidase (E.C.3.5.1.11), glutaryl-7-ACA acylase and D-amino acid oxidase (E.C.1.4.3.3),
**wherein**
the enzyme is immobilized on an aminofunctional organosiloxane polymer carrier material by covalent binding.

## Revendications

1. Enzyme fixée sur un support, choisie dans le groupe constitué par une pénicilline G-amidase (E.C. 3.5.1.11), une glutaryl-7-ACA-acylase et une D-aminoacide-oxydase (E.C. 1.4.3.3), fixée par liaison covalente à un support constitué d'un polymère d'organosiloxane à fonctions amino.

2. Enzyme fixée sur un support selon la revendication 1, caractérisée en ce que l'enzyme est fixée sur le support par l'intermédiaire d'un dialdéhyde.

3. Enzyme fixée sur un support selon la revendication 1 ou 2, caractérisée en ce que le rapport en masse de l'enzyme au support est compris entre 1 et 300 g de protéine par g de support humide.

4. Enzyme fixée sur un support selon l'une des revendications 1 à 3, caractérisée en ce que le support est sous forme de particules et a un diamètre moyen de particules de 0,01 à 3 mm.

5. Glutaryl-7-ACA-acylase fixée sur un support selon l'une des revendications 1 à 4, caractérisée en ce que le rapport en masse de l'enzyme au support est compris entre 10 et 110 mg de protéine par g de support solide.

6. Glutaryl-7-ACA-acylase fixée sur un support selon la revendication 5, caractérisée en ce que l'activité volumique spécifique est comprise entre 100 et 250 U par g de support humide.

7. D-aminoacide-oxydase fixée sur un support selon l'une des revendications 1 à 4, caractérisée en ce que le rapport en masse de l'enzyme au support est compris entre 5 et 25 mg de protéine par g de support humide.

8. D-aminoacide-oxydase fixée sur un support selon la revendication 7, qui est co-immobilisée avec une catalase (E.C. 1.11.1.6).

9. Utilisation d'enzymes fixées sur un support selon l'une des revendications 1 à 8, isolément ou en combinaison, dans une réaction de synthèse enzymatique.

10. Procédé d'amélioration de l'activité volumique d'enzymes fixées sur un support choisies dans le groupe constitué par une pénicilline G-amidase (E.C. 3.5.1.11), une glutaryl-7-ACA-acylase et une D-aminoacide-oxydase (E.C. 1.4.3.3), caractérisé en ce que l'on fixe l'enzyme par liaison covalente sur un support constitué par un polymère d'organosiloxane à fonctions amino.
